# FASCICULE DE BREVET EUROPEEN

(11) **EP 2 306 060 B1**
(45) Date de publication et mention de la délivrance du brevet: **16.05.2012**
(21) Numéro de dépôt: 09171832.0
(22) Date de dépôt: 30.09.2009
(51) Int. Cl.: F16L 29/02, F16L 37/40, F16L 37/60

(54) **Prise de distribution de fluide, notamment de gaz médical ou de vide**
Fluidverteilungsanschluss, insbesondere für medizinisches Gas oder Vakuum
Fluid distribution outlet, in particular for medical gas or vacuum

(43) Date de publication de la demande: 06.04.2011
(73) Titulaire: L'AIR LIQUIDE, Société Anonyme pour l'Etude et l'Exploitation des Procédés Georges Claude, 75007 Paris (FR); Flow Meter S.p.a., 24040 Levate (Bergamo) (IT); Air Liquide Sanità Service S.p.A., 20148 Milano (IT)
(72) Inventeur: Paratico, Roberto, 24040 Levate (BG) (IT); Tardieu, Christophe, 20121 Milano (IT)
(74) Mandataire: Pittis, Olivier

(56) Documents cités:
- EP-A1- 1 611 921
- EP-A1- 2 055 341
- FR-A1- 2 899 950
- US-A- 3 532 101
- US-A- 4 357 037
- "NF S 90-116: Prises muraleset fiches correspondantes pour fluides médicaux" juin 1988 (1988-06), AFNOR , La Plaine Saint-Denis , XP008121348 * le document en entier *

## Description

La présente invention concerne une prise de distribution de fluide et un ensemble prise/bloc de base auxquels peut se raccorder un appareillage médical destiné à être alimenté en gaz sous pression ou, à l'inverse, en vide, c'est-à-dire mis sous dépression.

Dans les salles de soins, les salles d'opération ou similaires au sein des bâtiments hospitaliers, tels les hôpitaux, les cliniques ou analogues, les personnels soignants doivent disposer de prises d'alimentation en gaz médical ou en vide de manière à pouvoir traiter le patient avec un gaz médical adéquat, tel l'oxygène, distribué par une prise d'oxygène et/ou procéder à des aspirations, notamment de fluides corporels, tel le sang, lors des interventions, grâce à une prise de vide.

Pour ce fait, il est usuel d'agencer des coffrets de raccordement, placés côte à côte, sur un des murs de la pièce ou sur une des parois mobiles d'un chariot, chaque coffret étant muni d'une prise délivrant un fluide donné, à savoir un gaz déterminé à usage médical ou du vide.

Lorsqu'un personnel soignant veut utiliser, un gaz ou du vide, il raccorde un appareillage médical au coffret délivrant le fluide en question. Ces coffrets de raccordement comprennent généralement une prise de fluide, qui représente la partie fixe d'un ensemble de branchement dont la partie mobile, c'est-à-dire un connecteur, est agencée à un appareillage à usage médical qui doit être alimenté avec ce gaz ou en vide.

Autrement dit, pour raccorder, de façon étanche, un appareillage à usage médical et un coffret de raccordement correspondant, il est généralement prévu un couplage mâle/femelle entre les parties fixe (prise) et mobile (connecteur de l'appareil), par exemple un couplage à enclenchement rapide à baïonnette d'un connecteur mobile (mâle) avec ladite prise fixe (femelle) du coffret ou boîtier.

La prise de fluide est constituée d'un corps de prise habituellement en acier ayant une extrémité distale filetée, pour être vissée de façon amovible et étanche sur un raccord du coffret de raccordement, et à l'opposé une extrémité proximale façonnée en écrou hexagonal pour pouvoir être serrée en vissage au moyen d'une clé de manoeuvre. Le corps de prise est traversé par un conduit de gaz ou passage principal, dans lequel est aménagé un siège de valve normalement fermé par un obturateur qui est constamment rappelé contre celui-là par des moyens élastiques.

À l'occasion d'un accouplement à enclenchement rapide, le connecteur de l'appareil médical ouvre la valve du corps de prise, en surmontant l'opposition des moyens élastiques, et il s'établit alors une communication de fluide entre le coffret de raccordement et l'appareillage médical, au travers du corps de prise. Le document FR2899950 montre une prise connue.

De tels prises et coffrets de raccordement doivent être de structure robuste et de fonctionnement sûr et ce, malgré la fréquence élevée à laquelle sont effectuées les opérations d'enclenchement et de déclenchement des connecteurs.

Cette dernière exigence nécessite de réaliser un entretien et des vérifications de l'intégrité structurelle et fonctionnelle de la prise de fluide et de tous ses composants à intervalles de temps réguliers. En particulier, il convient de procéder au remplacement de la partie interne du corps de prise, c'est-à-dire de l'obturateur et des moyens élastiques.

Or, cette opération entraîne des coûts tant pour le démontage et le remplacement de ces composants qu'en main d'oeuvre car elle nécessite l'intervention de personnel spécialisé chargé de la maintenance. En effet, un opérateur spécialisé doit d'abord retirer le corps de prise de la base du coffret de raccordement, avant de démonter l'obturateur et les moyens élastiques du corps de prise et de les remplacer, et enfin remonter le corps de prise sur la base du coffret de raccordement.

Les coûts de ces opérations périodiques sont relativement élevés, mais elles doivent être réalisées pour des raisons de sécurité et pour respecter les normes en vigueur.

Un problème qui se pose dans ce contexte est dès lors de pouvoir faciliter les opérations périodiques d'entretien grâce à une prise de fluide de structure améliorée pour coffret de raccordement d'une installation de distribution de fluide, à savoir de gaz comprimé ou de vide, permettant le raccordement d'un appareil à usage médical, comme par exemple un appareillage d'anesthésie, de respiration artificielle, d'aspiration et autres appareils similaires, et qui ait par ailleurs des caractéristiques structurelles et fonctionnelles propres à rendre extrêmement simples le montage et le démontage de la prise de gaz par rapport au coffret de raccordement.

Un autre but de l'invention est de proposer un coffret de raccordement pour une installation de distribution de fluide, munie d'une telle prise de distribution de fluide munie préférentiellement d'une connexion ou d'un branchement standard, apte à distribuer un gaz comprimé, tel un gaz médical, ou du vide utilisable lors de la mise en oeuvre d'une aspiration lors d'une intervention médicale ou autre.

Dans le cadre de l'invention, on désigne par le terme « fluide », un gaz ou un mélange gazeux, tel de l'oxygène, de l'air..., à une pression supérieure ou égale à la pression atmosphérique ou une mise en dépression, c'est-à-dire à une pression inférieure à la pression atmosphérique, encore appelée « vide ». La prise de fluide selon l'invention est donc utilisable pour alimenter un appareil médical en gaz sous pression ou en vide, par exemple pour réaliser une aspiration.

La solution de la présente invention est une prise de distribution de fluide comprenant:
- un corps de prise comprenant une extrémité distale filetée et une extrémité proximale conformée pour le branchement d'un connecteur d'un appareillage, ledit corps étant traversé par un passage principal de fluide reliant fluidiquement les extrémités proximale et distale;
- un siège de valve et un obturateur de valve mobile axialement étant aménagés dans ledit passage principal de fluide, ledit obturateur étant normalement repoussé vers le siège de valve par des moyens élastiques de manière à empêcher toute circulation de fluide dans ledit passage principal,
le passage principal de fluide traversant le corps est obturé au niveau de son extrémité distale, au moyen d'un capuchon de fermeture, ledit capuchon de fermeture étant traversé par au moins un passage de fluide et comportant, en outre, une partie en saillie se projetant vers l'extérieur, caractérisé en ce qu'au moins une partie de la périphérie externe du corps est enveloppée par un manchon et le corps de prise est muni à sa périphérie de nervurages s'étendant axialement coopérant avec des cannelures aménagées axialement sur la surface interne du manchon de manière à rendre le manchon et le corps de prise solidaires en rotation grâce à un couplage desdits nervurages avec lesdites cannelures.

Selon le cas, la prise de invention peut comprendre l'une ou plusieurs des caractéristiques suivantes :
- le passage principal de fluide traversant le corps comprend une embouchure, au niveau de son extrémité proximale, au sein de laquelle est fixé un insert comportant une tête située à l'extérieur du corps de prise et conformée pour le branchement d'un connecteur d'un appareillage, ladite tête comportant au moins un passage de fluide.
- la totalité du corps de la périphérie externe du corps est enveloppée par un manchon.
- le manchon enveloppant le corps de prise est fixé de façon irréversible au corps de prise.
- le corps et/ou le manchon sont réalisés en matière plastique.
- le manchon est fixé de façon irréversible au corps de prise au moyen d'une ou plusieurs languettes en partie malléables ou cassables, agencées dans la partie proximale du manchon et ayant une extrémité libre destinée à s'accrocher à un rebord du corps de prise. De préférence, sur le manchon est sérigraphée, la date de maintenance de la maintenance préventive à effectuer sur le corps de prise.
- l'insert et/ou les moyens élastiques sont en métal ou en alliage métallique.
- le corps de prise forme une structure monobloc avec la tête, le capuchon de fermeture et les moyens à valve comprenant le siège de valve, l'obturateur de valve et le ressort. De préférence, cette structure monobloc est sigillée et inviolable.

L'invention concerne également un ensemble de distribution de fluide formé par une prise selon l'invention fixée par vissage de son extrémité distale à un bloc de base de distribution de fluide alimenté par une canalisation d'alimentation en fluide.

Selon le cas, l'ensemble de l'invention peut comprendre l'une ou plusieurs des caractéristiques suivantes :
- le bloc de base pour gaz médicaux comporte une valve à sens unique et un obturateur de valve, ledit obturateur de valve étant repoussé par la partie en saillie du capuchon de fermeture de la prise, lorsque l'extrémité distale de la prise est complètement vissée sur le bloc de base.
- le manchon a une extrémité distale façonnée en accouplement de forme avec le bloc de base.
- l'extrémité distale du manchon comporte une découpe ayant un profil complémentaire de la forme externe du bloc de base.
- le bloc de base et la prise sont au moins partiellement logé dans boîtier fermé par un couvercle muni d'une ouverture au travers de laquelle fait saillie au moins une partie de l'extrémité proximale de la prise.

Un avantage de la prise selon la présente invention réside dans le fait que sa conception lui confère simplicité structurelle et coût réduit puisqu'elle permet d'obtenir une prise à usage unique pouvant être démontée facilement et remplacée en totalité, lors d'entretiens périodiques. Cet entretien est facilité par le manchon qui est clipsé de façon irréversible au corps de prise au moyen de languettes, en partie malléables ou cassables, prévues dans la partie proximale du manchon et ayant une extrémité libre destinée à s'accrocher par clipsage à un rebord du corps de prise.

D'autres caractéristiques et avantages de la prise de distribution de fluide selon la présente invention découleront de la description détaillée suivante, donnée à titre illustratif mais non limitatif, et faite en référence aux Figures annexées parmi lesquelles :

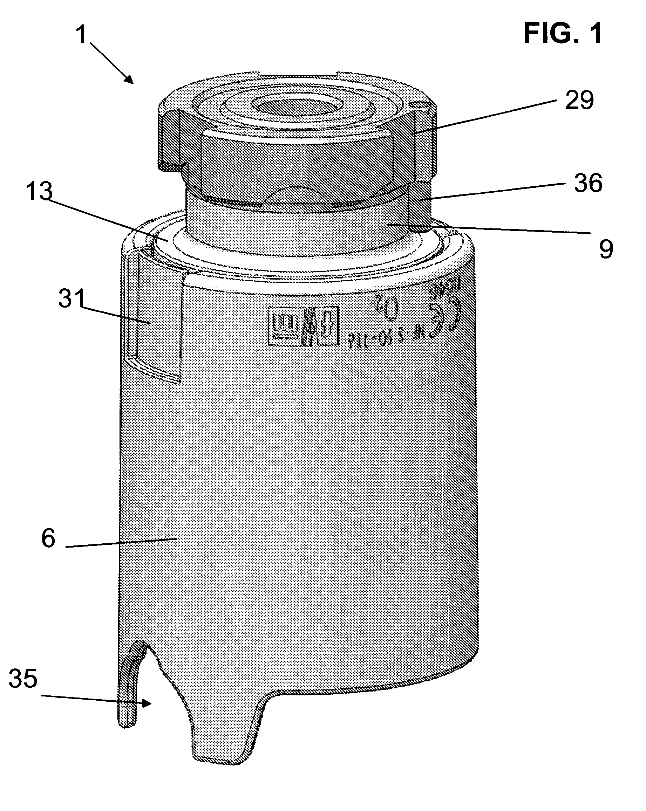

- la Figure 1 est une vue d'une prise de distribution de fluide selon la présente invention;

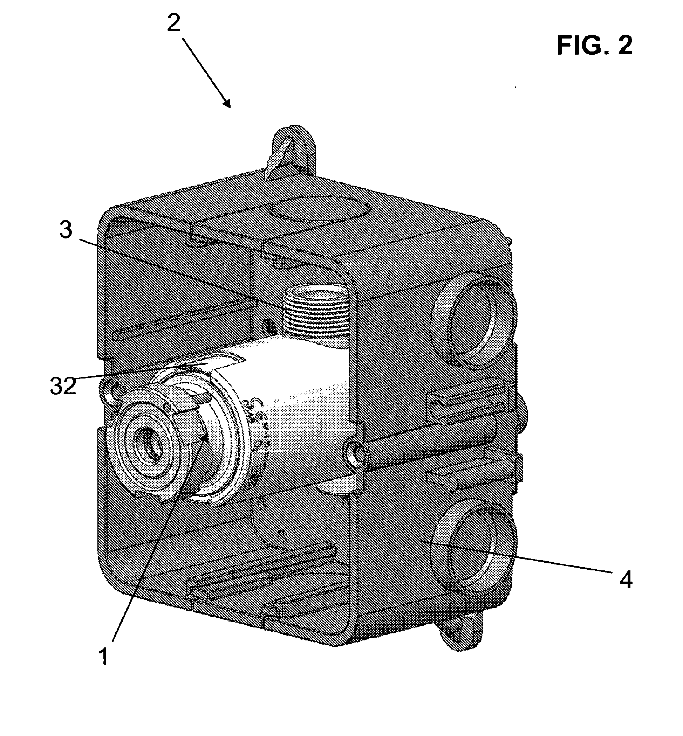

- la Figure 2 montre la prise de la Figure 1 installée dans un coffret de distribution de fluide;
- la Figure 3 est une vue partiellement éclatée de la prise de la Figure 1 avant son montage dans le coffret de raccordement ;

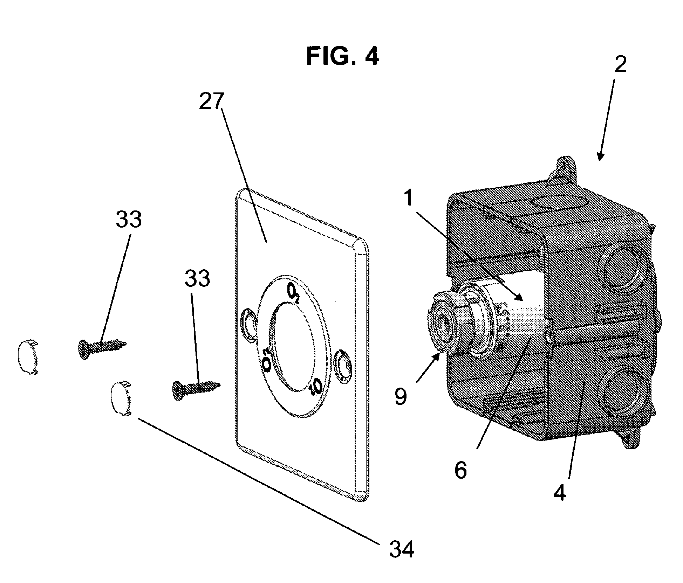

- la Figure 4 est une vue en perspective du coffret de raccordement de la Figure 2 équipée d'un couvercle de fermeture et identification;
- la Figure 5 est une vue éclatée de la prise de la Figure 1;
- la Figure 6 est une vue en coupe longitudinale de la prise de la Figure 1; et

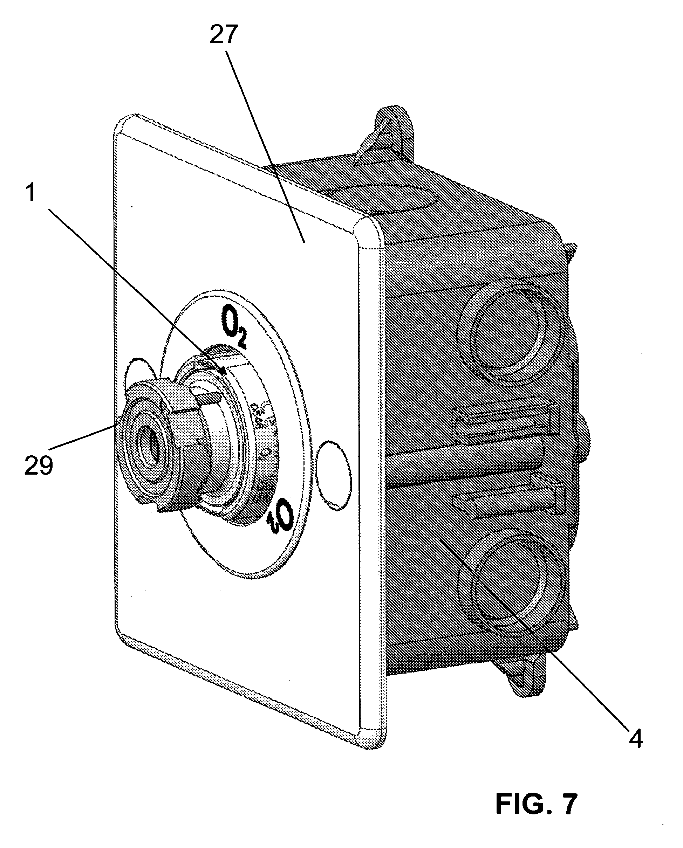

- la Figure 7 est une vue du coffret de raccordement de la Figure 4, après sa fermeture par le couvercle.

Une prise 1 de distribution de fluide, de préférence à raccordement standard, réalisée conformément à la présente invention est destinée à être montée dans un coffret de raccordement 2 d'une installation de distribution de gaz comprimé médical ou de vide apte à être connectée à un appareillage à usage médical.

Dans le cadre de l'invention, par le terme appareillage à usage médical, on entend par exemple un appareillage qui utilise un gaz déterminé à des fins thérapeutiques, un instrument chirurgical ou des appareils de même type, ou bien un appareillage technique hospitalier, c'est-à-dire un appareillage qui est utilisé non à des fins thérapeutiques, mais seulement en vue d'un diagnostic, ou encore pour le contrôle d'appareillages médicaux ou pour l'entraînement d'instruments médicaux et appareils de même type, ou bien qui exploite une dépression de vide pour une aspiration ou pour l'entraînement d'instruments médicaux et appareils de même type.

Pour simplifier l'exposé, la description qui suit est faite en se référant à un coffret de raccordement de distribution de gaz mais tout ce qui est décrit ci-après vaut également un coffret d'aspiration d'une installation de distribution de vide, étant donné que les analogies structurelles entre ces deux types de coffrets sont très fortes.

La prise 1 selon l'invention est donc destinée à recevoir le connecteur d'entrée d'un appareillage à usage médical qui utilise un gaz comprimé déterminé ou du vide, qui traverse la prise 1. Le connecteur associé à l'appareillage à usage médical n'est pas représenté sur les Figures car il est de type traditionnel, par exemple ce peut être un connecteur à baïonnette enfichable dans la prise 1 tel que décrit par exemple dans le document EP-A-2055341. Ce connecteur à enficher dans la prise 1 est préférentiellement de type normalisé, par exemple selon la norme française AFNOR NF-S 90-116.

Plus précisément, la prise 1 de l'invention comprend un corps de prise 7, encore appelé complément de prise, de forme essentiellement cylindrique et creux, c'est-à-dire que le corps 7 est traversé axialement par un passage principal 15 interne. Le corps 7 comporte par ailleurs une extrémité distale 8 munie d'un filetage 28 pour la fixation de la prise 1 sur un bloc 3, comme expliqué ci-après, et une extrémité proximale 9 munie d'une tête 29, par exemple façonnée en écrou hexagonal, comportant au moins un orifice central 38 pour le passage du fluide, sur laquelle vient se connecter le connecteur de l'appareillage médical.

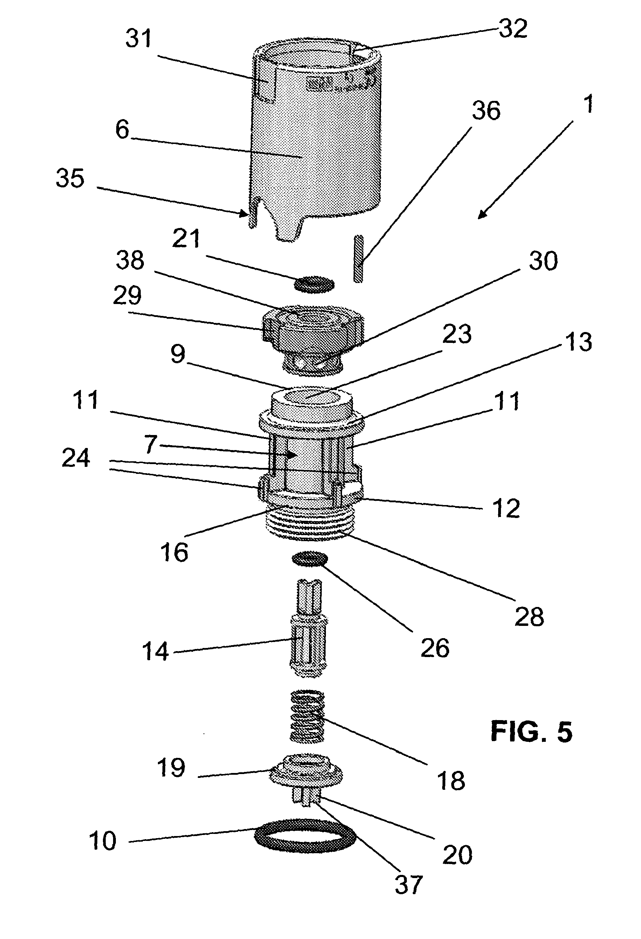

À la base du filetage 28, est aménagé un logement annulaire 16 pour recevoir une bague d'étanchéité 10 ou un joint, visible sur la Figure 5.

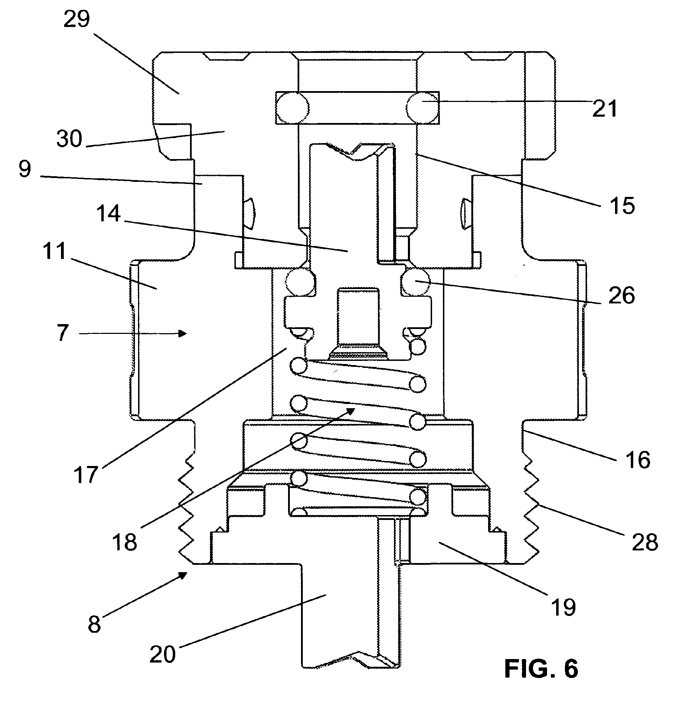

À l'intérieur du corps de prise 7 est aménagé le passage principal 15 de fluide qui est visible sur la coupe de la Figure 6, lequel débouche aux extrémités opposées du corps de prise 7, c'est-à-dire au niveau des extrémités proximale 9 et distale 8.

Dans le passage principal 15 est aménagé un siège 17 d'obturateur ou siège de valve coopérant avec un obturateur 14 mobile axialement dans le passage principal 15 de manière à contrôler le passage du fluide dans le corps 7. L'obturateur 14 est équipé d'un joint 26 d'étanchéité et de moyens élastiques 18, tel un ressort, qui agissent sur ledit obturateur 14 dans un sens tendant à le repousser en direction du siège 17, pour le maintenir en position de fermeture contre ledit siège 17 de valve et ainsi normalement empêcher le passage de fluide dans le corps 7.

Le corps de prise 7 est fermé à l'arrière, ou plus exactement au niveau de son extrémité distale 8, par un capuchon de fermeture 19 ou un bouchon perforé, c'est-à-dire traversé axialement par un ou plusieurs passages secondaires 37 de fluide. Le capuchon de fermeture 19 est rendu solidaire de l'extrémité distale 8 du corps de prise 7 par exemple au moyen d'un soudage par ultrasons ou tout autre moyen de solidarisation. Le capuchon de fermeture 19 est formé d'une seule pièce et comporte une partie en saillie 20 formant une pointe ou un doigt, se projetant axialement vers l'arrière, c'est-à-dire vers l'extérieur, et dont la fonction sera expliquée ci-après.

Les moyens élastiques 18, tel un ressort, sont logés dans le passage 17 et comprimés entre le capuchon de fermeture 19 et la paroi interne de l'obturateur 14.

Le corps de prise 7 comprend aussi un insert 30 introduit à travers l'embouchure 23 de l'extrémité proximale 9 du corps 7 et dont la tête façonnée 29 constitue la partie qui affleure à l'extérieur du corps de prise 7 et à laquelle vient se raccorder le connecteur ou raccord de l'appareillage médical. Plus précisément, l'insert 30 est de préférence formé d'une matière métallique et présente sur la tête 29 un bord périphérique qui est conformé pour offrir un accouplement de forme spécifique gaz avec un connecteur correspondant associé à l'appareillage à usage médical.

Une goupille 36 est insérée entre un orifice de la tête 29 et un siège du corps de prise 7 pour définir un point d'encliquetage prédéterminé entre les facettes de la tête façonnée 29 et les nervurages 11 du complément de prise 7. Par ailleurs, une bague d'étanchéité 21 faisant office de joint est agencée entre l'insert 30 et l'embouchure 23 du corps de prise 7.

Cet insert 30, la goupille d'encliquetage 36 et les moyens élastiques 18 sont préférentiellement des composants métalliques car ils sont soumis à une usure et à des efforts mécaniques. Par exemple l'insert 30 peut être réalisé en laiton.

Le coffret de raccordement 2 de l'installation de distribution de gaz comprimé ou de vide comprend quant à lui un bloc de base 3 logé à l'intérieur d'un boîtier 4 encastrable monté sur ledit mur. Le bloc de base 3 est un raccord terminal de l'installation de distribution de gaz affleurant sur le fond du boîtier encastrable 4.

Les boîtiers 4 encastrables sont généralement réalisées en une matière plastique de synthèse et sont fermés par un couvercle 27 de fermeture percé en son centre pour laisser dépasser l'extrémité proximale 9 et la tête façonnée 29 du corps de prise 7. Le couvercle 27 est vissé sur le boîtier 4 au moyen de vis 33 ou analogues, et des inserts circulaires 34 de finition sont clipsés dans les sièges logeant les têtes desdites vis 33.

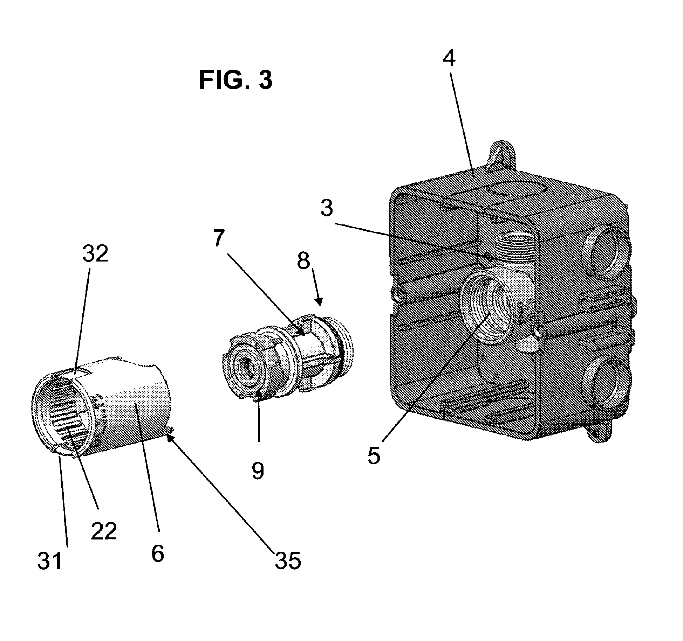

Comme illustré en Figure 3, le bloc de base 3 est essentiellement un raccord en forme de T avec un siège central 5 femelle, fileté intérieurement, destiné à recevoir une extrémité de la prise 1. Le bloc 3 est généralement réalisé en alliage métallique, par exemple en laiton.

Dans le siège central 5 du bloc de base 3 est généralement prévu un obturateur de valve (non représenté sur les figures), sauf dans la version pour vide où cet obturateur de valve n'est pas prévu.

Le bloc de base 3 en forme de T comprend donc en position centrale une valve à sens unique, normalement fermée, qui empêche le débit de gaz comprimé tant que le corps de prise 7 n'est pas complètement vissé et de façon étanche dans le siège 5, ce qui a pour effet simultané de commander l'ouverture de l'obturateur de valve du siège 5 à cause de la poussée axiale exercée sur l'obturateur par la partie en saillie 20 du capuchon de fermeture 19.

Après vissage de l'extrémité distale 8 du corps de prise 7 dans le siège 5 correspondant du bloc de base 3, la charge de fonctionner comme valve à sens unique passe de l'obturateur de valve du siège 5 du bloc 3 à l'obturateur 14 du siège de valve 17 du corps de prise 7.

Il est avantageux que le corps de prise 7 soit réalisé en matière plastique de synthèse, par exemple un techno-polymère comme le polycarbonate ou l'ABS, et puisse être vissé à la main dans le bloc de base 3. Dans ce but, le corps de prise 7 est muni à sa périphérie de nervurages 11 s'étendant longitudinalement, c'est axialement, sur au moins une partie de l'extension longitudinale du corps de prise 7.

La matière plastique formant le corps de prise 7 peut être pigmentée de manière à utiliser cette pigmentation comme un code couleur identifiant la prise toute entière ou une partie de celle-ci et, par ailleurs, le gaz débité par cette prise.

Comme détaillé en Figure 5, le corps de prise 7 présente des rebords annulaires 12 et 13, formant couronnes périphériques externes, entre lesquels s'étendent les nervurages 11. Le premier rebord 12 est situé à proximité du logement annulaire 16 et du filetage 28 tandis que le second rebord 13 est situé du côté opposé au précédent, à proximité de l'extrémité proximale du corps de prise 7.

Les nervurages 11 sont de préférence équidistants angulairement, par exemple à 90° l'un de l'autre, et comprennent des appendices ou ailettes 24 distales qui sont monobloc avec la partie distale des nervurages 11 et forment des saillies radiales. La fonction de ces ailettes 24 est expliquée ci-après.

Par ailleurs, il est prévu un manchon 6 enfilé coaxialement et irréversiblement sur la prise 1 selon l'invention, c'est-à-dire que le manchon 6 peut être solidarisé au corps de prise 7, par clipsage par exemple. Le manchon 6 est préférentiellement réalisé en matière plastique de synthèse, par exemple un techno-polymère comme le polycarbonate ou l'ABS. Le manchon 6 présente à l'intérieur une pluralité de cannelures 22 qui s'étendent parallèlement entre elles sur au moins une partie de l'extension longitudinale du manchon 6 même, à partir de son extrémité interne proximale, c'est-à-dire axialement.

De façon avantageuse, les ailettes 24 qui font saillie radialement des nervurages 11 du corps de prise 7 sont destinées à s'insérer chacune entre deux cannelures 22 contiguës du manchon 6 de façon à réaliser un accouplement cannelé entre le manchon 6 et le corps de prise 7. De cette façon, le manchon 6 est rendu solidaire en rotation du corps de prise 7 une fois que ledit corps de prise a été vissé sur le siège 5 du bloc de base 3.

Il est en outre prévu un enclenchement frontal entre le manchon 6 et le bloc de base 3 en forme de T. Plus précisément, l'extrémité distale du manchon 6 comporte une partie conformée 35 présentant dans l'ensemble une découpe orientée vers le bas, préférentiellement à profil arrondi, destinée à un accouplement de forme avec tout ou partie de la structure, généralement tubulaire, du bloc de base 3, à proximité du siège 5 dudit bloc 3.

En outre, partant de parties radialement opposées de l'extrémité proximale du manchon 6, on trouve des languettes 31, 32 rectangulaires, en partie malléables ou cassables, rendues solidaires du manchon 6 seulement le long d'un de leurs côtés courts et ayant le côté court opposé d'extrémité libre en forme de dent pour s'accrocher, de préférence par clipsage, sous le rebord 13 du corps de prise 7. Il est avantageux que ces languettes 31, 32 soient cassables quand est dépassée une force prédéterminée exercée par le technicien de maintenance pour libérer le corps de prise 7 afin de le remplacer. Il est avantageux que les languettes 31, 32 soient inviolables pour une personne non autorisée ou en tout cas non munie d'un outil spécifique.

Une fois que le corps de prise 7 a été vissé à la main dans le bloc de base 3 du coffret de raccordement 2 par un technicien de maintenance, ce dernier a la possibilité d'enfiler le manchon 6 sur le complément de prise 7 en faisant s'enclencher frontalement la découpe 35 avec le bout tubulaire du bloc de base 3. Cette action manuelle provoque simultanément un accouplement cannelé entre les ailettes 24 du corps de prise 7 et les cannelures 22 internes du manchon 6 qui rend solidaires en rotation le manchon 6 et le corps de prise 7.

En outre, le clipsage des languettes 31, 32 rend essentiellement irréversible l'accouplement cannelé entre le corps de prise 7 et le manchon 6 de telle sorte que la prise 1, une fois vissée dans le bloc de base 3, ne puisse plus se dévisser. Pour éviter que le corps de prise 7 ne se dévisse ou ne se desserre du bloc de base 3, il n'est plus nécessaire de le serrer à fond par vissage mais il suffit de le visser à la main, puisque la présence du manchon 6 empêche son dévissage.

Avantageusement, le corps de prise 7 et le manchon 6 peuvent être réalisés en matière à usage unique de type plastique de synthèse (techno polymère) et peuvent revêtir diverses couleurs, chacune correspondant par exemple à un type donné de gaz distribué par la prise considérée.

Il est avantageux que le manchon 6 et l'extrémité proximale du complément de prise 7 sortent du couvercle de fermeture 27 du boîtier 4 d'une distance suffisante, d'une part, pour permettre un accouplement correct avec le connecteur de l'appareillage médical et, d'autre part, pour recevoir des inscriptions identifiant le type de gaz débité par cette prise ou le lot de production de cette prise. De préférence, la prise comporte aussi des indications de lot, de date de péremption ....

Grâce à la conception de la prise selon la présente invention, un technicien de maintenance n'a qu'à démonter ou déboîter le manchon 6 et prélever le corps de prise, c'est-à-dire l'ensemble de la figure 6, en cassant les languettes 31, 32 et en le dévissant ensuite. De cette façon, tous les composants de la prise 1 peuvent être remplacés ou vérifiés selon une politique d'entretien programmé.

On peut aussi imaginer, comme autre mode de réalisation, de supprimer le façonnement en écrou hexagonal de la tête 29 de serrage, ce qui facilite ainsi le marquage du corps de prise 7, et d'employer des clés spécifiques pour l'assemblage au bloc de base 3 du coffret de raccordement.

Dans tous les cas, le corps de prise 7 forme donc un ensemble monobloc avec la tête 29, le capuchon de fermeture 19 et les moyens à valve, à savoir notamment le siège 17 de valve, l'obturateur 14 de valve et le ressort 18, c'est-à-dire que ces éléments constituent une sorte de cartouche extractible, pouvant être aisément insérée et solidarisée ou, à l'inverse, désolidarisée et extraite du bloc, lors des entretiens de la prise 1. Cet ensemble monobloc est préférentiellement à usage unique et est donc remplacé à chaque entretien de manière à garantir une prise en parfait état de fonctionnement.

Une prise et/ou un ensemble selon l'invention peuvent être utilisés pour contrôler la distribution d'un fluide, en particulier au sein d'un bâtiment hospitalier, de préférence un gaz, tel de l'oxygène, du N₂O ou de l'air médical, ou du vide. Désormais, la maintenance de la prise peut se faire facilement par échange standard de l'ensemble.

## Revendications

1. Prise (1) de distribution de fluide comprenant:
- un corps (7) de prise comprenant une extrémité distale (8) filetée et une extrémité proximale (9) conformée pour le branchement d'un connecteur d'un appareillage, ledit corps (7) étant traversé par un passage principal (15) de fluide reliant fluidiquement les extrémités proximale (9) et distale (8); et
- un siège de valve (17) et un obturateur (14) de valve mobile axialement étant aménagés dans ledit passage principal (15) de fluide, ledit obturateur (14) étant normalement repoussé vers le siège de valve (17) par des moyens élastiques (18) de manière à empêcher toute circulation de fluide dans ledit passage principal (15),
- le passage principal (15) de fluide traversant le corps (7) étant obturé au niveau de son extrémité distale (8), au moyen d'un capuchon de fermeture (19), ledit capuchon de fermeture (19) étant traversé par au moins un passage (37) de fluide et comportant, en outre, une partie en saillie (20) se projetant vers l'extérieur, **caractérisée en ce que** :
- au moins une partie de la périphérie externe du corps (7) est enveloppée par un manchon (6), et
- le corps de prise (7) est muni à sa périphérie de nervurages (11) s'étendant axialement coopérant avec des cannelures (22) aménagées axialement sur la surface interne du manchon (6) de manière à rendre le manchon (6) et le corps de prise (7) solidaires en rotation grâce à un couplage desdits nervurages (11) avec lesdites cannelures (22).

2. Prise selon la revendication 1, **caractérisée en ce que** le passage principal (15) de fluide traversant le corps (7) comprend une embouchure (23), au niveau de son extrémité proximale (9), au sein de laquelle est fixé un insert (30) comportant une tête (29) située à l'extérieur du corps de prise (7) et conformée pour le branchement d'un connecteur d'un appareillage, ladite tête (29) comportant au moins un passage (38) de fluide.

3. Prise selon l'une des revendications précédentes, **caractérisée en ce que** la totalité de la périphérie externe du corps (7) est enveloppée par le manchon (6).

4. Prise selon l'une des revendications précédentes, **caractérisée en ce que** le manchon (6) enveloppant le corps de prise (7) est fixé de façon irréversible au corps de prise (7).

5. Prise selon l'une des revendications précédentes, **caractérisée en ce que** le corps (7) et/ou le manchon (6) sont réalisés en matière plastique.

6. Prise selon l'une des revendications précédentes, **caractérisée en ce que** le manchon (6) est fixé de façon irréversible au corps de prise (1) au moyen d'une ou plusieurs languettes (31, 32) en partie malléables ou cassables, agencées dans la partie proximale du manchon (6) et ayant une extrémité libre destinée à s'accrocher à un rebord (13) du corps de prise (7).

7. Prise selon l'une des revendications précédentes, **caractérisée en ce que** l'insert (30) et/ou les moyens élastiques (18) sont en métal ou en alliage métallique.

8. Prise selon l'une des revendications précédentes, **caractérisée en ce que** le corps de prise (7) forme une structure monobloc avec la tête (29), le capuchon de fermeture (19) et les moyens à valve comprenant le siège (17) de valve, l'obturateur (14) de valve et le ressort (18).

9. Ensemble de distribution de fluide formé par une prise (1) selon l'une des revendications précédentes fixée par vissage de son extrémité distale (8) à un bloc de base (3) de distribution de fluide alimenté par une canalisation d'alimentation en fluide.

10. Ensemble selon la revendication 9, **caractérisé en ce que** le bloc de base (3) comporte une valve à sens unique et un obturateur de valve, ledit obturateur de valve étant repoussé par la partie en saillie (20) du capuchon de fermeture (19) de la prise (1), lorsque l'extrémité distale (8) de la prise (1) est complètement vissée sur le bloc de base (3).

11. Ensemble selon l'une des revendications 9 ou 10, **caractérisé en ce que** le manchon (6) a une extrémité distale (35) façonnée en accouplement de forme avec le bloc de base (3).

12. Ensemble selon l'une des revendications 9 à 11, **caractérisé en ce que** l'extrémité distale du manchon (6) comporte une découpe (35) ayant un profil complémentaire de la forme externe du bloc de base (3).

13. Ensemble selon l'une des revendications 9 à 12, **caractérisé en ce que** le bloc de base (3) et la prise (1) sont au moins partiellement logé dans boîtier (4) fermé par un couvercle (27) munie d'une ouverture au travers de laquelle fait saillie au moins une partie de l'extrémité proximale (9) de la prise (1).

14. Utilisation d'une prise (1) selon l'une des revendications 1 à 8 ou d'un ensemble selon l'une des revendications 9 à 13 pour contrôler la distribution d'un fluide, en particulier au sein d'un bâtiment hospitalier, de préférence un gaz ou un vide.

## Claims

1. Fluid distribution outlet (1) comprising:
- an outlet body (7) comprising a threaded distal end (8) and a proximal end (9) formed for the connection of an equipment connector, said body (7) having a main fluid passage (15) passing through it which provides a flow connection between the proximal (9) and distal (8) ends; and
- a valve seat (17) and an axially movable valve shutter (14) being provided in said main fluid passage (15), said shutter (14) normally being pushed back towards the valve seat (17) by resilient means (18) so as to prevent any circulation of fluid in said main passage (15),
- the main fluid passage (15) passing through the body (7) being blocked at its distal end (8) by means of a closure cap (19), said closure cap (19) having at least a fluid passage (37) passing through it and further comprising a protruding portion (20) which projects outwards,
**characterised in that**:
- at least a portion of the outer periphery of the body (7) is surrounded by a sleeve (6), and
- the outlet body (7) is equipped at its periphery with axially extending ribs (11) which cooperate with grooves (22) provided axially on the inner surface of the sleeve (6) in such a way that the sleeve (6) and the outlet body (7) are rotationally engaged owing to an interconnection between said ribs (11) and said grooves (22).

2. Outlet according to claim 1, **characterised in that** the main fluid passage (15) passing through the body (7) comprises, at its proximal end (9), a mouth (23) in which an insert (30) is fixed, which insert comprises a head (29) which is located outside the outlet body (7) and is formed for the connection of an equipment connector, said head (29) comprising at least a fluid passage (38).

3. Outlet according to either of the preceding claims, **characterised in that** the entirety of the outer periphery of the body (7) is surrounded by the sleeve (6).

4. Outlet according to any one of the preceding claims, **characterised in that** the sleeve (6) surrounding the outlet body (7) is irreversibly fixed to the outlet body (7).

5. Outlet according to any one of the preceding claims, **characterised in that** the body (7) and/or the sleeve (6) are made of plastics material.

6. Outlet according to any one of the preceding claims, **characterised in that** the sleeve (6) is irreversibly fixed to the outlet body (1) by means of one or more tabs (31, 32) which are malleable or breakable in part, arranged in the proximal portion of the sleeve (6) and have a free end for latching onto an edge (13) of the outlet body (7).

7. Outlet according to any one of the preceding claims, **characterised in that** the insert (30) and/or the resilient means (18) are made of metal or a metal alloy.

8. Outlet according to any one of the preceding claims, **characterised in that** the outlet body (7) forms a one-piece structure with the head (29), the closure cap (19) and the valve means comprising the valve seat (17), the valve shutter (14) and the spring (18).

9. Fluid distribution assembly formed by an outlet (1) according to any one of the preceding claims which is fixed, by screwing its distal end (8), to a fluid distribution base unit (3) which is fed by a fluid supply pipe.

10. Assembly according to claim 9, **characterised in that** the base unit (3) comprises a one-way valve and a valve shutter, said valve shutter being pushed back by the protruding portion (20) of the closure cap (19) of the outlet (1) when the distal end (8) of the outlet (1) is fully screwed on the base unit (3).

11. Assembly according to either claim 9 or claim 10, **characterised in that** the sleeve (6) has a distal end (35) which is shaped for positive connection to the base unit (3).

12. Assembly according to any one of claims 9 to 11, **characterised in that** the distal end of the sleeve (6) comprises a cut-out (35) having a profile complementary to the outer shape of the base unit (3).

13. Assembly according to any one of claims 9 to 12, **characterised in that** the base unit (3) and the outlet (1) are housed, at least in part, in a housing (4) closed by a lid (27) which is provided with an opening through which at least a portion of the proximal end (9) of the outlet (1) projects.

14. Use of an outlet (1) according to any one of claims 1 to 8 or of an assembly according to any one of claims 9 to 13 for controlling the distribution of a fluid, in particular in a hospital building, preferably a gas or a vacuum.

## Patentansprüche

1. Fluidverteilungsanschluss (1), umfassend:
- einen Anschlusskörper (7) mit einem distalen Ende (8), das mit Außengewinde versehen ist, und einem proximalen Ende (9), das für den Anschluss eines Verbinders einer Einrichtung versehen ist, wobei der Körper (7) von einem Hauptdurchlass (15) für Fluid durchsetzt ist, der das proximale Ende (9) und das distale Ende (8) fluidisch verbindet; und
- wobei einen Ventilsitz (17) und ein in axialer Richtung verschiebbares Ventilschließglied (14) in dem Hauptdurchlass (15) für Fluid angeordnet sind, wobei das Schließglied (14) normalerweise durch Federmittel (18) in Richtung des Ventilsitzes (17) zurückgedrückt wird, derart, dass jegliche Zirkulation von Fluid in den Hauptdurchlass (15) verhindert wird,
- wobei der Hauptdurchlass (15) für Fluid , der den Körper (7) durchsetzt, im Bereich seines distalen Endes (8) mit Hilfe einer Verschlusskappe (19) verschlossen ist, wobei die Verschlusskappe (19) von mindestens einem Durchlass (37) für Fluid durchsetzt ist und ferner einen auskragenden Teil (20) aufweist, der nach außen vorspringt,
**dadurch gekennzeichnet, dass**
- mindestens ein Teil des Außenumfangs des Körpers (7) von einer Hülse (6) umgeben ist und
- der Anschlusskörper (7) an seinem Umfang mit Rippen (11) versehen ist, die sich in axialer Richtung erstrecken und mit Rillen (22) zusammenwirken, die in axialer Richtung an der Innenfläche der Hülse (6) ausgebildet sind, derart, dass die Hülse (6) und der Anschlusskörper (7) dank einer Kopplung der Rippen (11) mit den Rillen (22) drehfest miteinander verbunden werden.

2. Anschluss nach Anspruch 1, **dadurch gekennzeichnet, dass** der Hauptdurchlass (15) für Fluid , der den Körper (7) durchsetzt, im Bereich seines proximalen Endes (9) eine Mündung (23) aufweist, in der ein Einsatz (30) befestigt ist, der einen Kopf (29) aufweist, der sich außerhalb des Anschlusskörpers (7) befindet und für den Anschluss eines Verbinders einer Einrichtung ausgebildet ist, wobei der Kopf (29) mindestens einen Durchlass (38) für Fluid aufweist.

3. Anschluss nach einem der vorhergehenden Ansprüche, **dadurch gekennzeichnet, dass** der gesamte Außenumfang des Körpers (7) von der Hülse (6) umgeben ist.

4. Anschluss nach einem der vorhergehenden Ansprüche, **dadurch gekennzeichnet, dass** die Hülse (6), die den Anschlusskörper (7) umgibt, auf irreversible Weise an dem Anschlusskörper (7) befestigt ist.

5. Anschluss nach einem der vorhergehenden Ansprüche, **dadurch gekennzeichnet, dass** der Körper (7) und/oder die Hülse (6) aus Kunststoff hergestellt sind.

6. Anschluss nach einem der vorhergehenden Ansprüche, **dadurch gekennzeichnet, dass** die Hülse (6) auf irreversible Weise an dem Anschlusskörper (1) mit Hilfe einer oder mehrerer zum Teil verformbarer oder brechbarer Zungen (31, 32) befestigt ist, die in dem proximalen Teil der Hülse (6) ausgebildet sind und ein freies Ende haben, das für das Einhängen an einem Bund (13) des Anschlusskörpers (7) vorgesehen ist.

7. Anschluss nach einem der vorhergehenden Ansprüche, **dadurch gekennzeichnet, dass** der Einsatz (30) und/oder die Federmittel (18) aus Metall oder Metalllegierung sind.

8. Anschluss nach einem der vorhergehenden Ansprüche, **dadurch gekennzeichnet, dass** der Anschlusskörper (7) mit dem Kopf (29), der Verschlusskappe (19) und den Ventilmitteln, die den Ventilsitz (17), das Ventilschließglied (14) und die Feder (18) umfassen, eine einstückige Struktur bildet.

9. Fluidverteilungsanordnung, die von einem Anschluss (1) nach einem der vorhergehenden Ansprüche gebildet ist, der durch Verschraubung seines distalen Endes (8) an einem Basisblock (3) zur Fluidverteilung befestigt ist, der durch eine Fluidzuleitung versorgt wird.

10. Anordnung nach Anspruch 9, **dadurch gekennzeichnet, dass** der Basisblock (3) ein Einwegventil und ein Ventilschließglied aufweist, wobei das Ventilschließglied durch den auskragenden Teil (20) der Verschlusskappe (19) des Anschlusses (1) zurückgedrückt wird, wenn das distale Ende (8) des Anschlusses (1) vollständig an dem Basisblock (3) verschraubt ist.

11. Anordnung nach einem der Ansprüche 9 oder 10, **dadurch gekennzeichnet, dass** die Hülse (6) ein distales Ende (35) hat, das für die formschlüssige Verbindung mit dem Basisblock (3) geformt ist.

12. Anordnung nach einem der Ansprüche 9 bis 11, **dadurch gekennzeichnet, dass** das distale Ende der Hülse (6) einen Ausschnitt (35) mit einem Profil aufweist, das zu der äußeren Form des Basisblocks (3) komplementär ist.

13. Anordnung nach einem der Ansprüche 9 bis 12, **dadurch gekennzeichnet, dass** der Basisblock (3) und der Anschluss (1) mindestens teilweise in einem Gehäuse (4) gelagert sind, das durch einen Deckel (27) verschlossen ist, der mit einer Öffnung versehen ist, durch die mindestens ein Teil des proximalen Endes (9) des Anschlusses (1) vorspringt.

14. Verwerdung eines Anschlusses (1) nach einem der Ansprüche 1 bis 8 oder einer Anordnung nach einem der Ansprüche 9 bis 13 zur Kontrolle der Verteilung eines Fluid, insbesondere in einem Krankenhausgebäude, vorzugsweise eines Gases oder eines Vakuums.
